# EUROPÄISCHE PATENTSCHRIFT

(11) **EP 2 110 162 B1**
(45) Veröffentlichungstag und Bekanntmachung des Hinweises auf die Patenterteilung: **25.05.2011**
(21) Anmeldenummer: 09155084.8
(22) Anmeldetag: 13.03.2009
(51) Int. Cl.: A61N 5/10

(54) **Medizinische Anlage und Verfahren zum Docken einer Positioniervorrichtung an ein Shuttle**
Medicinal assembly and method for docking a positioning device to a shuttle
Installation médicale et procédé d'amarrage d'un dispositif de positionnement sur une navette

(30) Priorität: 16.04.2008 DE 102008019114
(43) Veröffentlichungstag der Anmeldung: 21.10.2009
(73) Patentinhaber: Siemens Aktiengesellschaft, 80333 München (DE)
(72) Erfinder: Hüttenberger, Stefan, 91054, Erlangen (DE); Rietzel, Eike, 64289, Darmstadt (DE); Saar, Matthias, 96146, Altendorf (DE)

(56) Entgegenhaltungen:
- EP-A- 1 238 685
- EP-A- 1 749 550
- WO-A-2009/036169
- KATUIN J E ET AL: "The use of industrial robot arms for high precision patient positioning" APPLICATION OF ACCELERATORS IN RESEARCH AND INDUSTRY, XX, XX, 1. November 2002 (2002-11-01), Seiten 1-4, XP002456988

## Beschreibung

Die Erfindung betrifft eine medizinische Anlage, in welcher ein vorbereiteter Patient oder Qualitätssicherungs-Phantome auf einem Shuttle positioniert werden, und in welcher eine Positioniervorrichtung an das Shuttle dockt. Weiterhin betrifft die Erfindung ein Verfahren zum Docken einer Positioniervorrichtung an ein Shuttle. Eine derartige medizinische Anlage findet insbesondere in der Strahlentherapie bzw. Partikeltherapie Einsatz, da hier Patienten üblicherweise in einem Vorbereitungsraum vorbereitet werden, mithilfe des Shuttle in einen Untersuchungs- oder Behandlungsraum gefahren werden und anschließend über eine Positioniervorrichtung, die an das Shuttle dockt, in eine gewünschte Position positioniert werden.

Die Partikeltherapie ist ein etabliertes Verfahren zur Behandlung von Gewebe, insbesondere von Tumorerkrankungen. Partikel, vornehmlich Ionen wie Protonen, Kohlenstoffionen, oder andere Ionensorten, werden auf hohe Energien in einem Beschleuniger beschleunigt, zu einem Partikelstrahl geformt und anschließend in einem Behandlungsraum auf das zu bestrahlende Gewebe gerichtet. Die Partikel geben ihrer Energie in einem relativ umschriebenen Bereich innerhalb des Zielvolumens ab.

Es ist vergleichsweise aufwändig und kostenintensiv, Partikel auf die notwendige Energie zu beschleunigen und zu einem Strahl zu formen. Um eine Partikeltherapieanlage dennoch effektiv zu betreiben, wird üblicherweise ein Behandlungsraum der Partikeltherapieanlage lediglich für einen konkreten Bestrahlungsvorgang oder ein Untersuchungsraum der Partikeltherapieanlage lediglich für das Durchführen einer Untersuchung - beispielsweise mit einem Computertomographen - genutzt.

Dies bedeutet, dass eine Vorbereitung oder eine Nachbereitung des Patienten üblicherweise in einem getrennten, hierfür vorgesehenen Raum durchgeführt wird. Nach erfolgreicher Vorbereitung, beispielsweise durch Immobilisierung des Patienten auf einer Patientenliege in einem eigens dafür vorgesehenen Immobilisierungsraum, wird die Patientenliege mit einem so genannten mobilen Shuttle ihn die jeweiligen Räume für eine Behandlung bzw. Untersuchung gefahren. Nach durchgeführter Untersuchung bzw. Behandlung wird der Patient wieder sofort aus dem Untersuchungs- bzw. Behandlungsraum gefahren, so dass diese Räume für einen nächsten Patienten zur Verfügung stehen.

Ein mobiles Shuttle umfasst dabei üblicherweise eine Patientenhalterungsvorrichtung - beispielsweise eine Patientenliege oder einen Patientenstuhl - und eine mobile Tragevorrichtung, auf der die Patientenhalterungsvorrichtung gelagert werden kann, so dass das mobile Shuttle von einem Raum in einen anderen gefahren werden kann.

Eine Möglichkeit sieht vor, in einem Untersuchungsraum bzw. in einem Behandlungsraum den vorbereiteten Patienten auf der Patientenhalterungsvorrichtung zu belassen und die Patientenhalterungsvorrichtung selbst in dem Untersuchungsraum bzw. in dem Behandlungsraum mithilfe einer Positioniervorrichtung passend zu positionieren, beispielsweise über einen Roboterarm.

Alternativ können auch QA-Phantome (QA für engl.: "quality assurance", Qualitätssicherung) auf einer Haltevorrichtung gelagert und von einer Tragevorrichtung abgenommen werden. Zur Durchführung der QA-Maßnahme kann der Roboterarm die Halterungsvorrichtung mit den QA-Phantomen abnehmen und im Raum entsprechend positionieren.

Um ein korrektes Zusammenspiel zwischen dem Roboterarm und dem Shuttle zu gewähren, ist es bisher notwendig, das Shuttle bzw. die Halterungsvorrichtung manuell genau zu positionieren und darauf zu achten, dass das Shuttle oder ein Teil des Shuttle - wie die Halterungsvorrichtung oder die Tragevorrichtung - nicht versehentlich verschoben wird.

Die nachveröffentlichte WO 2009/036169 A offenbart ein Verfahren, bei dem die Position eines Shuttle mithilfe eines visuellen Docking-Systems ermittelt wird.

Es ist daher die Aufgabe der Erfindung, eine medizinische Anlage bereitzustellen, in der ein Dockvorgang einer Positioniervorrichtung an ein Shuttle sicher durchführbar ist bei gleichzeitig hohem Maß an Flexibilität und Komfort. Weiterhin ist es die Aufgabe der Erfindung, ein Verfahren zum Docken der Positioniervorrichtung an ein Shuttle anzugeben, das ein hohes Maß an Sicherheit bei gleichzeitig hohem Komfort und hoher Flexibilität ermöglicht.

Die Aufgabe der Erfindung wird gelöst durch eine medizinische Anlage nach Anspruch 1 sowie durch ein Verfahren nach Anspruch 10. Vorteilhafte Weiterbildungen finden sich in den Merkmalen der abhängigen Ansprüche.

Demnach umfasst die medizinische Anlage einen Raum, in welchem ein Shuttle angeordnet ist. Das Shuttle umfasst dabei eine Halterungsvorrichtung und eine Tragevorrichtung für die Halterungsvorrichtung. Auf der Halterungsvorrichtung können z.B. ein Patient oder eines oder mehrere QA-Phantome gelagert werden. In dem Raum ist eine Positioniervorrichtung angeordnet, die dazu ausgebildet ist, an das Shuttle anzudocken, wodurch die Halterungsvorrichtung von der Tragevorrichtung abgenommen bzw. auf die Tragevorrichtung aufgeladen werden kann und gegebenenfalls in eine vorbestimmte Lage gebracht werden kann.

Weiterhin ist ein Positionserkennungssystem vorhanden, mit der die räumliche Position des Shuttle oder Teile des Shuttle im Raum ermittelbar ist. Eine Steuerungseinheit, mit der die Positioniervorrichtung gesteuert werden kann, ist derart ausgebildet, dass die Positioniervorrichtung beim Docken eines Shuttle mithilfe der ermittelten Position insbesondere automatisch gesteuert wird.

Insbesondere kann die medizinische Anlage als eine Partikeltherapieanlage ausgebildet sein. Der Raum der medizinischen Anlage kann beispielsweise ein Behandlungsraum der Partikeltherapieanlage sein oder ein Untersuchungsraum, in dem beispielsweise eine Computertomographie zur Untersuchung der räumlichen Lage der zu bestrahlenden Organe durchgeführt wird.

Ein Dockvorgang der Positioniervorrichtung an das Shuttle wird folglich dann durchgeführt, wenn die Positioniervorrichtung z.B. die Halterungsvorrichtung greift und von der Tragevorrichtung des Shuttle abnimmt, d.h. wenn die Positioniervorrichtung an die Halterungsvorrichtung dockt. Ein Dockvorgang wird auch dann durchgeführt, wenn die Halterungsvorrichtung, die durch die Positioniervorrichtung bereits ergriffen ist, wieder auf die Tragevorrichtung des Shuttle gelegt wird. Anschließend kann die Positioniervorrichtung von der Halterungsvorrichtung wieder getrennt werden (abdocken). Insbesondere kann das Shuttle selbst Teil der medizinischen Anlage sein.

Üblicherweise ist das Shuttle mobil, d.h. dass die Tragevorrichtung mobil ausgebildet ist, sodass das Shuttle insgesamt, d.h. die mobile Tragevorrichtung und die Halterungsvorrichtung als Ganzes verfahren werden kann, z.b. von einem Raum in einen anderen Raum.

Diese mobile Ausgestaltung des Shuttle ist jedoch nicht zwingend notwendig. Beispielsweise kann das Shuttle auch eine ortsfeste Tragevorrichtung und eine Halterungsvorrichtung, die abnehmbar auf der ortsfesten Tragevorrichtung gelagert ist, umfassen. Die ortsfeste Tragevorrichtung befindet sich in der Nähe der Positioniervorrichtung, so dass die Positioniervorrichtung an das Shuttle docken kann und die Halterungsvorrichtung von der Positioniervorrichtung abnehmen bzw. wieder aufladen kann. Diese Ausgestaltung ist beispielsweise für Halterungsvorrichtungen denkbar, auf der QA-Phantome angeordnet sind. Eine Halterungsvorrichtung mit QA-Phantomen kann nach Durchführung der QA-Maßnahmen wieder auf eine ortsfeste Tragevorrichtung - beispielsweise eine an der Wand angeordnete, regalartige Vorrichtung - abgelegt werden. Auch hier kann es geschehen, dass ein unbeabsichtigtes Verrücken der Halterungsvorrichtung auf der Tragevorrichtung passiert, so dass eine gewünschte Position der Halterungsvorrichtung im Raum nicht immer gegeben ist.

Mithilfe des Positionserkennungssystems wird die Sicherheit und der Komfort beim Docken der Positioniervorrichtung an das Shuttle deutlich erhöht. Wenn z.B. ein mobiles Shuttle in den Raum der Anlage gefahren wird, muss das Shuttle nicht exakt im Raum positioniert werden, um den weiteren Ablauf zu ermöglichen. Auch bei einem nicht exakt positionierten Shuttle erkennt das System die Position des Shuttle und steuert die Positioniervorrichtung entsprechend. Sollte sich während einer Untersuchung/Behandlung die Position der mobilen Tragevorrichtung ändern - z.B. durch ungewolltes Anstoßen oder manuelles Verschieben -, wird diese Änderung entsprechend registriert und beim erneuten Ablegen der Halterungsvorrichtung auf der mobilen Tragevorrichtung berücksichtigt. Auch bei einer ortsfesten Tragevorrichtung und einer ungenau abgelegten Halterungsvorrichtung auf der ortsfesten Tragevorrichtung funktioniert das Positionserkennungssystem in analoger Weise. Eine über das Positionserkennungssystem hinausgehende Hardware ist nicht nötig. Manuelle Interventionsschritte werden auf ein Minimum reduziert.

Die exakte Interaktion der Positioniervorrichtung mit dem Shuttle ermöglicht zu dem ein weitgehend erschütterungsfreies Abnehmen/Aufladen der Halterungsvorrichtung. Hierdurch erhöht sich die Patientensicherheit, da insbesondere bei einer Strahlentherapie Erschütterungen eines Patienten zu einer ungewünschten Verlagerung des Patienten führen können.

Falls ermittelt wird, dass sich das Shuttle außerhalb der Reichweite der Positioniervorrichtung befindet, kann ein Warnsignal ausgegeben werden. Ein Anwender kann daraufhin das Shuttle in eine für die Positioniervorrichtung günstigere Position bringen.

In einer Ausführungsvariante ist die Positioniervorrichtung als ein Roboterarm - z.B. als ein sechsachsiger Knickarmroboter - ausgebildet, wodurch die Bewegungsmöglichkeit und damit die Flexibilität der Positioniervorrichtung deutlich erhöht wird.

In einer vorteilhaften Ausführungsvariante ist das Positionserkennungssystem als ein externes Positionserkennungssystem, insbesondere zur kontaktlosen Ermittlung der Position des Shuttle ausgebildet. Das Positionserkennungssystem kann in einem Deckenbereich des Raumes angeordnet sein, da von hier aus aufgrund der günstigen Abstände zwischen Positionserkennungssystem und Shuttle besonders einfach die Position eines in den Raum gefahrenen Shuttle überwacht und ermittelt werden kann.

Das Positionserkennungssystem kann dabei als ein elektromagnetisches Positionserkennungssystem ausgebildet sein, d.h. als ein Positionserkennungssystem, das mithilfe von elektromagnetischen Signalen, insbesondere mithilfe zumindest eines RFID-Transponders (RFID für engl.: "Radio Frequency Identification"), den räumlichen Ort des Shuttle ermittelt.

Alternativ und/oder zusätzlich kann das Positionserkennungssystem als ein optisches Positionserkennungssystem ausgebildet sein. Eine Ortung des Shuttle kann dann beispielsweise über ein Lasersystem - z.B. über ein dreidimensionales Laserscannersystem - erfolgen. Denkbar sind aber auch optische Kameras, mit denen Bilder des Shuttle aufgenommen und ausgewertet werden und anschließend der Ort des Shuttle bestimmt wird. Beispielsweise kann auch ein Oberflächen detektierendes Kamerasystem eingesetzt werden.

In dem Raum kann zumindest eine erste Markierung raumfest angeordnet werden, die durch das Positionserkennungssystem detektierbar ist. Die Position des Shuttle kann daraufhin in Bezug auf die erste raumfeste Markierung ermittelt werden. Die genaue Lokalisation des Shuttle kann mit dieser ortsfesten Referenz erleichtert werden, eine Justierung des Positionserkennungssystems vereinfacht werden.

Weiterhin kann das Positionserkennungssystem derart ausgebildet sein, dass die Position des Shuttle über zumindest eine zweite Markierung, die an dem Shuttle selbst angeordnet ist, ermittelt werden kann. Eine Markierung ermöglicht eine einfachere automatische Lokalisierung des Shuttle. Beispielsweise kann eine Markierung an Halterungsvorrichtung angeordnet sein und/oder auch andere Tragevorrichtung, insbesondere wenn die Tragevorrichtung mobil ist.

Bei der Verwendung mehrerer Markierungen, raumfest und/oder am Shuttle angeordnet, kann die Lokalisation des Shuttle redundant durchgeführt werden, wodurch sich die Sicherheit und Genauigkeit erhöht. Es können auch verschiedenartige Erkennungssysteme zusammen eingesetzt werden, so dass die Lokalisation durch die verschiedenen Erkennungssysteme redundant auf unterschiedliche Weise ermittelt werden kann.

In einer vorteilhaften Ausführungsvariante kann das Positionserkennungssystem derart ausgebildet sein, dass mithilfe einer zusätzlichen Kodierung neben der Position des Shuttle auch ein funktioneller Betriebszustand und/oder ein Typ des Shuttle - z.B. der Typ der Tragevorrichtung und/oder der Typ der Halterungsvorrichtung - ermittelt werden kann. Auf diese Weise kann beispielsweise festgestellt werden, ob bei einem mobilen Shuttle die Bremse angezogen oder gelöst ist, ob die Halterungsvorrichtung mit der Tragevorrichtung verriegelt oder gelöst ist, etc. Die Steuerung der Positioniervorrichtung kann entsprechend dem zusätzlich ermittelten funktionellen Betriebszustand und/oder dem Typ durchgeführt werden. Auf diese Weise kann so verhindert werden, dass die Halterungsvorrichtung von der Tragevorrichtung abgenommen wird, wenn festgestellt wird, dass die Halterungsvorrichtung mit der Tragevorrichtung verriegelt ist oder dass sich bei einem mobilen Shuttle das Shuttle noch in einem ungebremsten Zustand befindet. Auf diese Weise kann die Steuerung der Positioniervorrichtung in Abhängigkeit des speziellen Typus der Halterungsvorrichtung durchgeführt werden, falls beispielsweise mehrere unterschiedliche Typen von Halterungsvorrichtungen in der medizinischen Anlage eingesetzt werden, die jeweils ein geringfügig unterschiedliches Docken der Positioniervorrichtung erfordern.

Das Verfahren zum Docken einer Positioniervorrichtung an ein Shuttle, wobei das Shuttle eine Halterungsvorrichtung und eine Tragevorrichtung für die Halterungsvorrichtung umfasst, weist folgende Schritte auf:
- Anordnen des Shuttle in einen Raum einer medizinischen Anlage,
- Ermitteln der Position des Shuttle über ein Positionserkennungssystem,
- Steuern der Positioniervorrichtung derart, dass beim Docken der Positioniervorrichtung an das Shuttle die ermittelte Position des Shuttle verwendet wird.

In vorteilhafter Weise wird die Position des Shuttle mithilfe zumindest einer ersten Markierung, die raumfest angeordnet ist, und/oder mithilfe zumindest einer zweiten Markierung, die an der Halterungsvorrichtung angeordnet ist, und/oder mithilfe zumindest einer dritten Markierung, die an der Tragevorrichtung angeordnet ist, ermittelt.

Zusätzlich zu der Position des Shuttle kann in vorteilhafter Weise durch das Positionserkennungssystem ein funktioneller Betriebszustand und/oder ein Typ des Shuttle ermittelt werden und hierauf basierend die Steuerung der Positioniervorrichtung angepasst werden.

In besonders vorteilhafter Weise kann das Verfahren zusätzlich derart weitergebildet werden, dass ein Warnsignal ausgegeben wird, falls bei der Positionsermittlung festgestellt wird, dass sich das Shuttle außerhalb einer Reichweite der Positioniervorrichtung befindet.

Weiterbildungen, wie sie bei der medizinischen Anlage näher beschrieben worden sind, können auch bei dem Verfahren Anwendung finden und umgekehrt.

Ausführungsformen der Erfindung mit vorteilhaften Weiterbildungen gemäß den Merkmalen der abhängigen Ansprüche werden anhand der folgenden Zeichnung näher erläutert, ohne jedoch darauf beschränkt zu sein. Es zeigen:
- Fig. 1: einen schematischen Überblick über eine Partikelthera- pieanlage,
- Fig. 2: eine Ansicht eines Behandlungsraums einer Partikelthe- rapieanlage mit einer robotischen Positioniervorrich- tung,
- Fig. 3: eine Aufsicht auf ein Shuttle mit Markierungen, die am Shuttle und im Bodenbereich angeordnet sind, und
- Fig. 4: und 5 jeweils eine schematische Übersicht über einzel- ne Verfahrensschritte, die bei Dockvorgängen der Posi- tioniervorrichtung an das Shuttle durchgeführt werden.

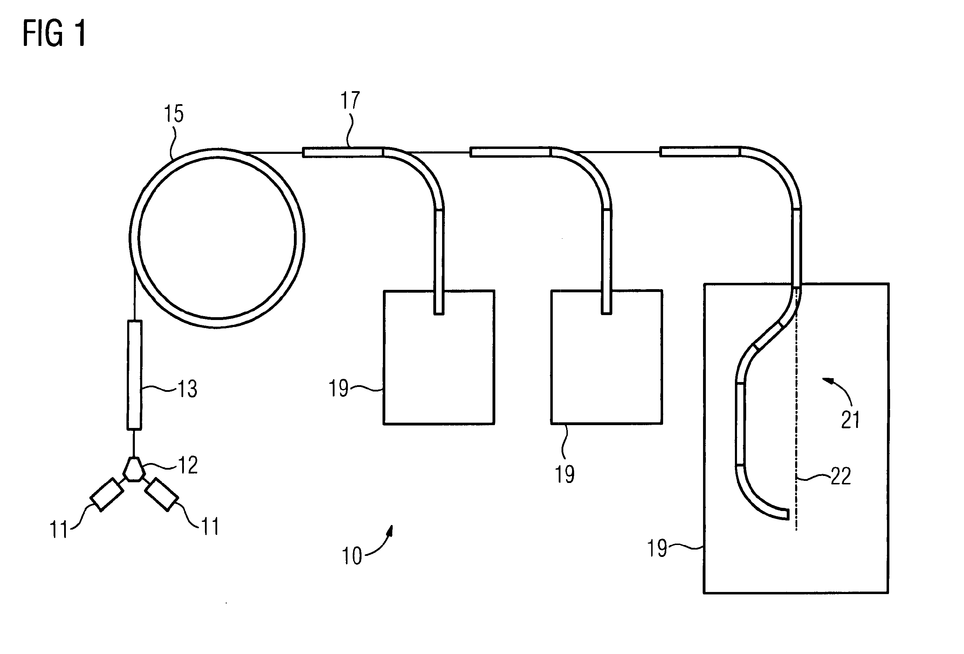

Fig. 1 zeigt einen schematischen Überblick über den Aufbau einer Partikeltherapieanlage 10. In einer Partikeltherapieanlage 10 erfolgt insbesondere eine Bestrahlung eines Körpers, insbesondere eines tumorerkrankten Gewebes, mit einem Partikelstrahl.

Als Partikel werden vornehmlich Ionen wie beispielsweise Protonen, Pionen, Heliumionen, Kohlenstoffionen oder andere Ionensorten eingesetzt. Üblicherweise werden derartige Partikel in einer Partikelquelle 11 erzeugt. Wenn, wie in Figur 1 dargestellt, zwei Partikelquellen 11 vorhanden sind, die zwei verschiedene Ionensorten erzeugen, kann zwischen diesen beiden Ionensorten innerhalb eines kurzen Zeitintervalls umgeschaltet werden. Dazu wird beispielsweise ein Schaltmagnet 12 verwendet, der zwischen den Ionenquellen 11 einerseits und einem Vorbeschleuniger 13 andererseits angeordnet ist. Z.B. kann hierdurch die Partikeltherapieanlage 10 mit Protonen und mit Kohlenstoffionen gleichzeitig betrieben werden.

Die von der oder einer der Ionenquellen 11 erzeugten und gegebenenfalls mit dem Schaltmagneten 12 ausgewählten Ionen werden in dem Vorbeschleuniger 13 auf ein erstes Energieniveau beschleunigt. Der Vorbeschleuniger 13 ist beispielsweise ein Linearbeschleuniger (LINAC für engl.: "LINear ACcelerator"). Anschließend werden die Partikel in einen Beschleuniger 15, beispielsweise ein Synchrotron oder Zyklotron, eingespeist. In dem Beschleuniger 15 werden sie auf hohe Energien, wie sie zur Bestrahlung nötig sind, beschleunigt. Nachdem die Partikel den Beschleuniger 15 verlassen, führt ein Hochenergiestrahl-Transportsystem 17 den Partikelstrahl zu einem oder mehreren Behandlungsräume 19. In einem Behandlungsraum 19 werden die beschleunigten Partikel auf einen zu bestrahlenden Körper gerichtet. Je nach Ausgestaltung erfolgt dies von einer festen Richtung (in so genannten "fixed beam"-Räumen) aus oder aber über eine um eine Achse 22 bewegliche rotierbare Gantry 21 von verschiedenen Richtungen aus.

Der anhand der Figur 1 dargestellte Aufbau einer Partikeltherapieanlage 10 ist im Stand der Technik bekannt und typisch für viele Partikeltherapieanlagen, kann aber auch hiervon abweichen.

Die nachfolgend beschriebenen Ausführungsbeispiele der Erfindung können in derartigen oder ähnlichen Partikeltherapieanlagen Einsatz finden oder aber auch allgemein in medizinischen Anlagen.

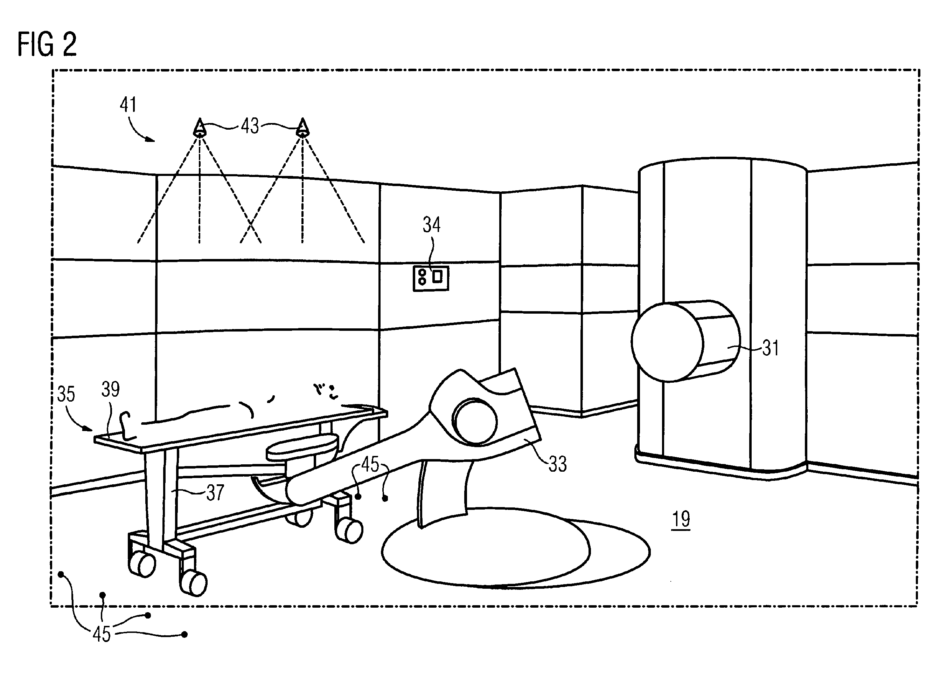

Fig. 2 zeigt eine Ansicht eines Behandlungsraums 19 in einer Partikeltherapieanlage. In einem derartigen Behandlungsraum 19 kann eine Bestrahlung eines Patienten mit einem Partikelstrahl erfolgen, der - wie hier gezeigt - aus einem festen Strahlaustritt 31 austreten kann. Die korrekte Positionierung des Patienten in Bezug auf den Partikelstrahl wird von einer robotischen Positioniervorrichtung 33 - hier gezeigt als sechsachsiger Knickarmroboter - übernommen. Die Steuerung der robotischen Positioniervorrichtung 33 wird durch eine Steuerungseinheit 34 übernommen.

Die Vorbereitung des Patienten findet üblicherweise in einem anderen Raum statt. Der für eine Bestrahlungssitzung vorbereitete Patient wird mit einem Shuttle 35 in den Behandlungsraum 19 gefahren. Das Shuttle 35 umfasst dabei eine mobile Tragevorrichtung 37 und einer auf der mobilen Tragevorrichtung 37 gelagerte und abnehmbare Patientenhalterungsvorrichtung - hier dargestellt als Patientenliege 39. Die Vorbereitung des Patienten kann alternativ auch im Behandlungsraum 19 selbst durchgeführt werden.

Nachdem sich der vorbereitete Patient auf dem Shuttle 35 im Behandlungsraum 19 befindet, wird die Patientenliege 39 durch die Positioniervorrichtung 33 von der mobilen Tragevorrichtung 37 abgenommen und in gewünschter Weise im Raum positioniert. Nach erfolgter Bestrahlung wird die Patientenliege 39 wiederum mithilfe der Positioniervorrichtung 33 auf der mobilen Tragevorrichtung 37 abgeladen.

Um ein einfaches, sicheres und genaues Ergreifen der Patientenliege 39 durch die Positioniervorrichtung 33 zu ermöglichen, ist an der Decke im Behandlungsraum 19 ein Positionserkennungssystem 41 angeordnet. Mithilfe dieses Positionserkennungssystems 41 kann die räumliche Lage des Shuttle 35 genau bestimmt werden und hierauf basierend eine Steuerung der Positioniervorrichtung 33 bei Dockvorgängen durchgeführt werden. Das Positionserkennungssystem 41 kann - wie hier dargestellt - zwei Detektoren 43 umfassen, mit denen die Position des Shuttle 35 redundant ermittelt werden kann.

Das Positionserkennungssystem 41 kann dabei erste Markierungen 45, die sich im Bodenbereich des Behandlungsraums 19 befinden, detektieren, sowie gegebenenfalls Markierungen, die am Shuttle 35 angeordnet sind.

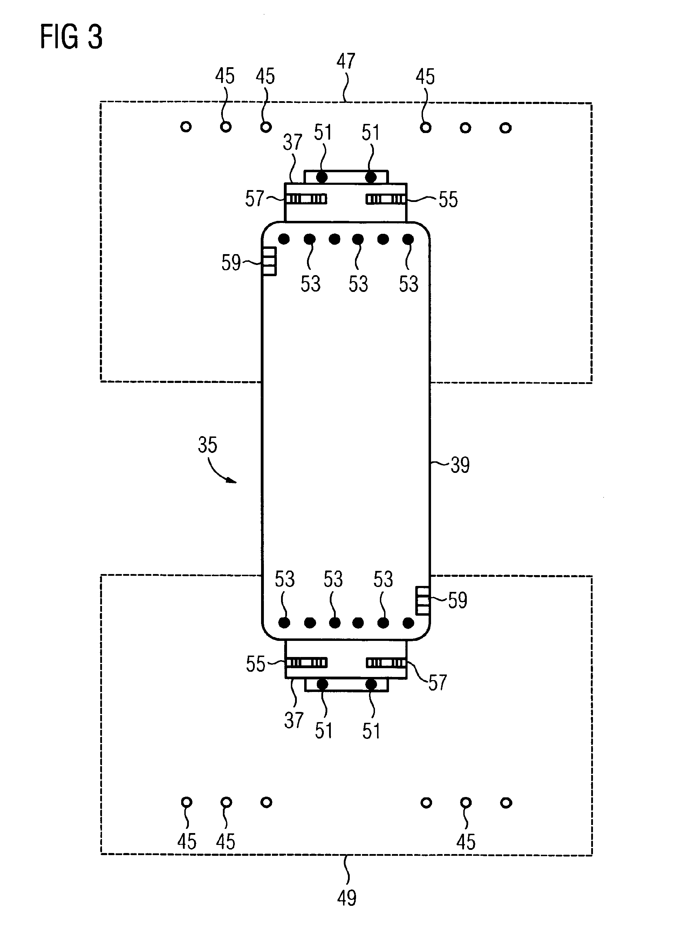

Anhand von Fig. 3 wird dieses Konzept näher erläutert. Gezeigt ist eine Aufsicht auf ein Shuttle 35 mit einer Patientenliege 39. Das Shuttle 35 wird ungefähr in dem Bereich abgestellt, in dem die Patientenliege 39 durch die Positioniervorrichtung 33 ergriffen werden kann. Daraufhin scannt das Positionserkennungssystem 41 sowohl den Kopfbereich 47 als auch den Fußbereich 49 des Shuttle 35. Dabei kann einer der Detektoren 43 für den Kopfbereich 47 zuständig sein und der andere der Detektoren 43 für den Fußbereich 49.

Als räumliche Referenz dient die räumliche Lage von raumfesten, im Bodenbereich angeordneten ersten Markierungen 45. Weiterhin wird die räumliche Lage von zweiten Markierungen 51, die an der mobilen Tragevorrichtung 37 sowohl im Kopfbereich 47 als auch im Fußbereich 49 angeordnet sind, sowie die räumliche Lage von dritten Markierungen 53, die an der Patientenliege 39 im Kopf- als auch im Fußbereich 47, 49 angeordnet sind, ermittelt.

Die räumliche Lage der zweiten Markierungen 51 und der dritten Markierungen 53 kann zu der räumlichen Lage der raumfesten, ersten Markierungen 45 in Beziehung gesetzt werden, also verrechnet werden, und so die exakte Position des Shuttle 35 im Raum ermittelt werden.

Darüber hinaus sind am Shuttle 35 erste Kodierungen 55 angeordnet, die kennzeichnen, ob sich das Shuttle in einem gebremsten Zustand befindet oder nicht. Weiterhin sind zweite Kodierungen 57 am Shuttle 35 angeordnet, die kennzeichnen, ob die Patientenliege 39 mit der mobilen Tragevorrichtung 37 verriegelt ist oder nicht. Die erste Kodierungen 55 und die zweiten Kodierungen 57 können ebenfalls durch das Positionserkennungssystem 41 erkannt werden.

Zudem können dritte Kodierungen 59 z.B. an der Patientenliege 39 angeordnet sein, mit denen das Positionserkennungssystem 41 den Typen der Patientenliege 39 und/oder den Typen der mobilen Tragevorrichtung 37 ermitteln kann. Dies ist vorteilhaft, wenn in der medizinischen Anlage verschiedene Arten von Shuttle 35 eingesetzt werden, die jeweils ein leicht unterschiedliches Docken der Positioniervorrichtung 33 erfordern.

Wie hier dargestellt, können sämtliche Markierungen/Kodierungen 45, 51, 53, 55, 57, 59 doppelt vorhanden sein und über die zwei Detektoren 43 des Positionserkennungssystems 41 redundant erkannt werden, wodurch sich die Sicherheit erhöht. Dies ist jedoch nicht zwingend notwendig.

Nachdem ermittelt worden ist, dass sich das Shuttle 35 in Reichweite der Positioniervorrichtung 33 befindet, und nachdem ermittelt worden ist, dass sich das Shuttle 35 in gebremsten Zustand befindet und dass die Patientenliege 39 nicht mit der mobilen Tragevorrichtung 37 verriegelt ist, kann die Positioniervorrichtung 33 zur Abnahme der Patientenliege 39 von der mobilen Tragevorrichtung 37 gesteuert werden. Falls eine der genannten Bedingungen nicht vorliegt, kann ein Warnsignal abgegeben werden, so dass ein Anwender die fehlende Bedingung beheben kann. Der Dockvorgang kann daraufhin starten und anhand der ermittelten Position und gegebenenfalls anhand des ermittelten Typs der Patientenliege gesteuert werden.

In analoger Weise wird verfahren, wenn die Patientenliege 39 wieder auf die mobile Tragevorrichtung 37 gelegt werden soll. Die Position und gegebenenfalls der funktionelle Betriebszustand der mobilen Tragevorrichtung 37 bzw. der Typ des Shuttle 35 werden durch das Positionserkennungssystem 43 detektiert und die Positioniervorrichtung 33 mit der Patientenliege 39 entsprechend gesteuert.

Die hier anhand eines Behandlungsraums 19 beschriebenen Ausführungen gelten in analoger Weise für einen Untersuchungsraum, bei dem beispielsweise ein vorbereiteter Patient über die Positioniervorrichtung in einem Computertomographen für eine Computertomographie-Untersuchung in geeigneter Weise positioniert wird.

Als Positionserkennungssystem 41 können verschiedene Ausführungen eingesetzt werden. Bei einem elektromagnetischen Positionserkennungssystem können als Markierungen bzw. Kodierungen beispielsweise elektromagnetisch arbeitende Marker wie z.B. RFID-Transponder eingesetzt werden. Optische Systeme können beispielsweise mit einem Laserscannersystem, z.B. einem dreidimensionalen Laserscannersystem, ausgestattet werden. Als Markierungen/Kodierungen können durch Laserstrahlen detektierbare Marker eingesetzt werden. Wenn als optisches System eine Kamera wie eine Videokamera oder eine Oberflächen detektierende Kamera eingesetzt wird, können die Markierungen/Kodierungen so ausgestaltet sein, dass sie in einem aufgenommenen Bild z.B. durch automatische Bildanalyseverfahren detektiert werden. Denkbar sind auch Systeme mit Ultraschallsensoren zur Ortung von entsprechenden Markern.

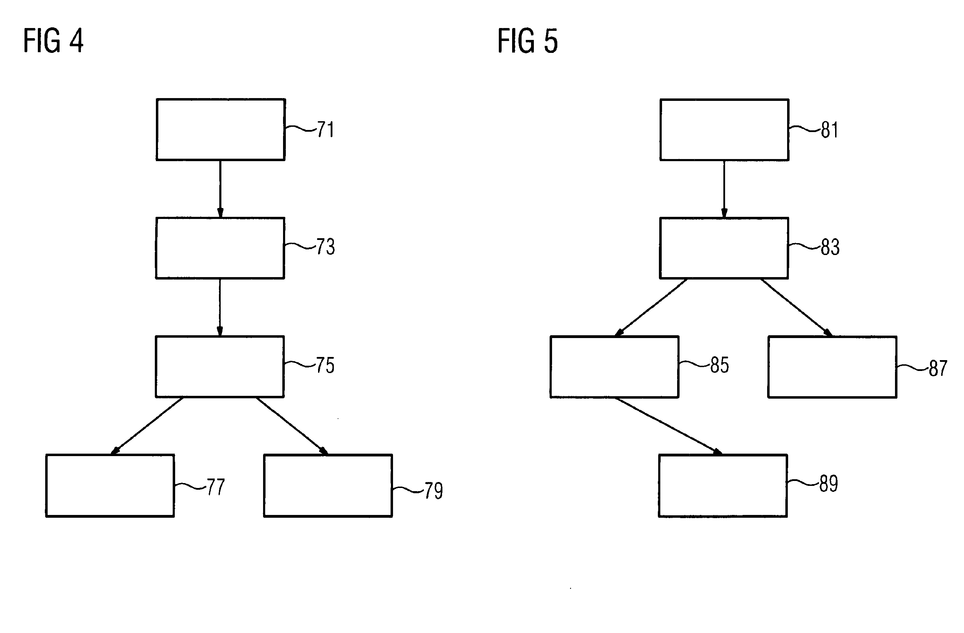

Fig. 4 zeigt eine schematische Übersicht über einzelne Verfahrensschritte, wie sie beim Abnehmen der Patientenhalterungsvorrichtung von der mobilen Tragevorrichtung ausgeführt werden können. Zunächst wird das Shuttle in den Raum gefahren und ungefähr in dem Bereich abgestellt, in dem die Positioniervorrichtung an das Shuttle docken kann (Schritt 71). Daraufhin wird die räumliche Position des Shuttle ermittelt (Schritt 73). Weiterhin wird ein funktioneller Betriebszustand und/oder ein Typ des Shuttle ermittelt, beispielsweise ein gebremster/ungebremster Zustand der mobilen Tragevorrichtung, eine Verriegelung/Entriegelung zwischen Patientenhalterungsvorrichtung und mobiler Tragevorrichtung oder ähnliches (Schritt 75). Wenn sich das Shuttle an geeignetem Ort befindet und der funktionelle Betriebszustand es erlaubt, erfolgt ein Docken der Positioniervorrichtung an das Shuttle und ein Abnehmen der Patientenhalterungsvorrichtung von der mobilen Tragevorrichtung (Schritt 77). Andernfalls wird ein Warnsignal ausgegeben (Schritt 79).

Fig. 5 zeigt eine schematische Übersicht über einzelne Verfahrensschritte, wie sie beim Aufladen der Patientenhalterungsvorrichtung auf die mobile Tragevorrichtung ausgeführt werden können. Nach Beendigung der Behandlung/Untersuchung wird die Position der mobilen Tragevorrichtung erneut ermittelt (Schritt 81). Weiterhin wird geprüft, ob sich die mobile Tragevorrichtung bzw. Patientenhalterungsvorrichtung in einem funktionellen Betriebszustand befinden, der es erlaubt, eine Patientenhalterungsvorrichtung auf der mobilen Tragevorrichtung abzulegen; gegebenenfalls wird auch der Typ der mobilen Tragevorrichtung bzw. der Patientenhalterungsvorrichtung ermittelt (Schritt 83). Wenn sich die mobile Tragevorrichtung an geeignetem Ort befindet und wenn ein geeigneter funktioneller Betriebszustand vorliegt, erfolgt ein Docken der Positioniervorrichtung mit der Patientenhalterungsvorrichtung an die mobile Tragevorrichtung (Schritt 85). Andernfalls wird ein Warnsignal abgegeben (Schritt 87). Nach erfolgreicher Aufladung der Patientenhalterungsvorrichtung auf der mobilen Tragevorrichtung kann das Shuttle wieder aus dem Raum gefahren werden (Schritt 89).

Anhand von Fig. 2 bis Fig. 5 wurde eine Ausführungsform der Erfindung erläutert, bei der ein mobiles Shuttle mit einer mobilen Tragevorrichtung sowie eine als Patientenhalterungsvorrichtung ausgebildeten Halterungsvorrichtung zum Einsatz kommt. Andere Ausführungsformen sehen vor, dass anstelle einer Patientenhalterungsvorrichtung eine Halterungsvorrichtung für QA-Phantome eingesetzt werden kann. In diesem Fall wird die Halterungsvorrichtung mit darauf angeordneten QA-Phantomen durch die Positioniervorrichtung an einer gewünschten Position im Raum positioniert und die entsprechende QA-Maßnahme durchgeführt. Weiterhin kann anstelle des mobilen Shuttles auch ein ortsfestes Shuttle im Bestrahlungsraum eingesetzt werden. Hier kann beispielsweise eine ortsfeste Tragevorrichtung, z.B. eine regalartige Konstruktion, eine durch die Positioniervorrichtung abnehmbare Halterungsvorrichtung tragen. Auch in diesen und anderen Ausführungsformen kann das beschriebene Positionserkennungssystem zum einfachen und sicheren Docken der Positioniervorrichtung an das Shuttle eingesetzt werden.

## Patentansprüche

1. Medizinische Anlage mit einem Raum (19), in welchem ein Shuttle (35) mit einer Halterungsvorrichtung (39) und einer Tragevorrichtung (37) für die Halterungsvorrichtung (39) anordenbar ist, die medizinische Anlage umfassend:
- eine in dem Raum (19) angeordnete Positioniervorrichtung (33), die ausgebildet ist zum Docken an die Halterungsvorrichtung (39) des Shuttle (35) derart, dass die Halterungsvorrichtung (39) von der Tragevorrichtung (37) abnehmbar bzw. auf die Tragevorrichtung (37) aufladbar ist,
- eine Steuerungseinheit (34) zur Steuerung der Positioniervorrichtung (33),
- ein Positionserkennungssystem (41, 43) zur Ermittlung der räumlichen Position des Shuttle (35) im Raum (19),
wobei die Steuerungseinheit (34) derart ausgebildet ist, die Positioniervorrichtung (33) beim Docken an das Shuttle (35) mithilfe der ermittelten Position zu steuern, **dadurch gekennzeichnet, dass**
das Positionserkennungssystem (41, 43) in einem Deckenbereich des Raumes (19) angeordnet ist.

2. Medizinische Anlage nach Anspruch 1, wobei die Positioniervorrichtung (33) als ein Roboterarm, insbesondere als ein sechsachsiger Knickarmroboter, ausgebildet ist.

3. Medizinische Anlage nach Anspruch 1 oder 2, wobei das Positionserkennungssystem (41, 43) als ein externes Positionserkennungssystem zur kontaktlosen Ermittlung der Position des Shuttle (35) ausgebildet ist.

4. Medizinische Anlage nach einem der Ansprüche 1 bis 3, wobei
das Positionserkennungssystem (41, 43) als ein elektromagnetisches Positionserkennungssystem ausgebildet ist, insbesondere zur räumlichen Ortung des Shuttle (35) mithilfe zumindest eines RFID-Transponders.

5. Medizinische Anlage nach einem der Ansprüche 1 bis 3, wobei
das Positionserkennungssystem (41, 43) als ein optisches Positionserkennungssystem ausgebildet ist.

6. Medizinische Anlage nach einem der Ansprüche 1 bis 5, wobei
in dem Raum (19) zumindest eine erste Markierung (45) raumfest angeordnet ist und das Positionserkennungssystem (41, 43) derart ausgebildet ist, dass die Position des Shuttle (35) in Bezug auf die erste raumfeste Markierung (45) ermittelbar ist.

7. Medizinische Anlage nach einem der Ansprüche 1 bis 6, wobei
das Positionserkennungssystem (41, 43) derart ausgebildet ist, die Position des Shuttle (35) über zumindest eine weitere Markierung (51, 53), die an dem Shuttle (35) angeordnet ist, zu ermitteln.

8. Medizinische Anlage nach einem der Ansprüche 1 bis 7, wobei
das Positionserkennungssystem (41, 43) derart ausgebildet ist, mithilfe einer Kodierung (55, 57, 59) zusätzlich zu der Position des Shuttle (35) einen funktionellen Betriebszustand und/oder einen Typen des Shuttle (35) zu ermitteln.

9. Verfahren zum Docken einer Positioniervorrichtung (33) an ein Shuttle (35), wobei das Shuttle (35) eine Halterungsvorrichtung (39) und eine Tragevorrichtung (37) für die Halterungsvorrichtung (39) umfasst, das Verfahren aufweisend folgende Schritte:
- Anordnen des Shuttle(35) in einen Raum (19) einer medizinischen Anlage (10),
- Ermitteln der Position des Shuttle (35) über ein Positionserkennungssystem (41, 43),
- Steuern der Positioniervorrichtung (33) derart, dass beim Docken der Positioniervorrichtung (33) an das Shuttle (35) die ermittelte Position des Shuttle (35) verwendet wird, **dadurch gekennzeichnet, dass**
das Positionserkennungssystem (41, 43) in einem Deckenbereich des Raumes (19) angeordnet ist.

10. Verfahren nach Anspruch 9, wobei
die Position des Shuttle (35) mithilfe zumindest einer ersten Markierung (45), die raumfest angeordnet ist, und/oder mithilfe zumindest einer zweiten Markierung (51), die an der Tragevorrichtung (37) angeordnet ist, und/oder mithilfe zumindest einer dritten Markierung (53), die an der Halterungsvorrichtung (39) angeordnet ist, ermittelt wird.

11. Verfahren nach Anspruch 9oder 10, wobei
durch das Positionserkennungssystem (41, 43) zusätzlich zu der Position des Shuttle (35) ein funktioneller Betriebszustand und/oder ein Typ des Shuttle (35) ermittelt wird und die Steuerung der Positioniervorrichtung (33) hierauf basierend angepasst wird.

12. Verfahren nach einem der Ansprüche 9 bis 11, wobei
ein Warnsignal ausgegeben wird, falls bei der Positionsermittlung festgestellt wird, dass sich das Shuttle (35) außerhalb einer Reichweite der Positioniervorrichtung (33) befindet.

## Claims

1. Medical facility having a room (19), in which a shuttle (35) comprising a support device (39) and a carrying device (37) for the support device (39) may be disposed, the medical facility comprising:
- a positioning device (33) disposed in the room (19) and designed for docking with the support device (39) of the shuttle (35) in such a way that the support device (39) is removable from the carrying device (37) and/or loadable onto the carrying device (37),
- a control unit (34) for controlling the positioning device (33),
- a position detection system (41, 43) for determining the spatial position of the shuttle (35) in the room (19),
wherein the control unit (34) is designed so as to control the positioning device (33) during docking with the shuttle (35) with the aid of the determined position **characterized in that**
the position detection system (41, 43) is arranged in a ceiling area of the room (19).

2. Medical facility according to claim 1, wherein the positioning device (33) is embodied as a robot arm, in particular a six-axis buckling arm robot.

3. Medical facility according to claim 1 or 2, wherein the position detection system (41, 43) is embodied as an external position detection system for contactless determination of the position of the shuttle (35).

4. Medical facility according to one of claims 1 to 3,
wherein
the position detection system (41, 43) is embodied as an electromagnetic position detection system, in particular for spatial localization of the shuttle (35) with the aid of at least one RFID transponder.

5. Medical facility according to one of claims 1 to 3,
wherein
the position detection system (41, 43) is embodied as an optical position detection system.

6. Medical facility according to one of claims 1 to 5,
wherein
in the room (19) at least one first marker (45) is disposed in a room-fixed manner and the position detection system (41, 43) is designed in such a way that the position of the shuttle (35) may be determined in relation to the first room-fixed marker (45).

7. Medical facility according to one of claims 1 to 6,
wherein
the position detection system (41, 43) is designed so as to determine the position of the shuttle (35) by means of at least one further marker (51, 53) that is disposed on the shuttle (35).

8. Medical facility according to one of claims 1 to 7,
wherein
the position detection system (41, 43) is designed so as to determine in addition to the position of the shuttle (35) a functional operating state and/or a type of the shuttle (35) with the aid of a code (55, 57, 59).

9. Method of docking a positioning device (33) with a shuttle (35), wherein the shuttle (35) comprises a support device (39) and a carrying device (37) for the support device (39), the method comprising the following steps:
- dispose the shuttle (35) in a room (19) of a medical facility (10),
- determine the position of the shuttle (35) by means of a position detection system (41, 43),
- control the positioning device (33) in such a way that during docking of the positioning device (33) with the shuttle (35) the determined position of the shuttle (35) is used.

10. Method according to claim 9, wherein
the position of the shuttle (35) is determined with the aid of at least one first marker (45), which is disposed in a room-fixed manner, and/or with the aid of at least one second marker (51), which is disposed on the carrying device (37), and/or with the aid of at least one third marker (53), which is disposed on the support device (39).

11. Method according to claim 9 or 10, wherein
by means of the position detection system (41, 43) in addition to the position of the shuttle (35) a functional operating state and/or a type of the shuttle (35) is determined and on the basis thereof the control of the positioning device (33) is adapted.

12. Method according to one of claims 9 to 11, wherein a warning signal is emitted if during determination of the position it is established that the shuttle (35) is situated outside of a range of the positioning device (33).

## Revendications

1. Installation médicale comprenant un local (19), dans lequel peut être disposée une navette (35) pourvue d'un dispositif support (39) et d'un dispositif porteur (37) du dispositif support (39), ladite installation médicale comprenant :
- un dispositif de positionnement (33) placé dans le local (19) et conçu pour être amarré sur le dispositif support (39) de la navette (35) de telle façon que le dispositif support (39) puisse être retiré du dispositif porteur (37) respectivment rechargé sur le dispositif porteur (37),
- une unité de commande (34) destinée à commander le dispositif de positionnement (33),
- un système de reconnaissance de position (41, 43) destiné à détecter la position dans l'espace de la navette (35) dans le local (19),
l'unité de commande (34) étant conçue pour commander le dispositif de positionnement (33) lors de l'amarrage sur la navette (35) à l'aide de la position détectée,
**caractérisée en ce que**
le système de reconnaissance de position (41, 43) est placé dans une zone de plafond du local (19).

2. Installation médicale selon la revendication 1,
le dispositif de positionnement (33) étant réalisé sous la forme d'un bras de robot, en particulier sous la forme d'un robot à bras articulé à six axes.

3. Installation médicale selon la revendication 1 ou 2,
le système de reconnaissance de position (41, 43) étant réalisé sous la forme d'un système externe de reconnaissance de position, destiné à la détection sans contact de la position de la navette (35).

4. Installation médicale selon l'une des revendications 1 à 3, le système de reconnaissance de position (41, 43) étant réalisé sous la forme d'un système électromagnétique de reconnaissance de position, destiné en particulier à la localisation dans l'espace de la navette (35) à l'aide d'au moins un transpondeur RFID.

5. Installation médicale selon l'une des revendications 1 à 3, le système de reconnaissance de position (41, 43) étant réalisé sous la forme d'un système optique de reconnaissance de position.

6. Installation médicale selon l'une des revendications 1 à 5, au moins un premier repère (45) étant placé à un endroit fixe dans le local (19), et le système de reconnaissance de position (41, 43) étant conçu de telle façon que la position de la navette (35) puisse être détectée par rapport au premier repère fixe (45).

7. Installation médicale selon l'une des revendications 1 à 6, le système de reconnaissance de position (41, 43) étant conçu de manière à détecter la position de la navette (35) par l'intermédiaire d'au moins un autre repère (51, 53), lequel est placé sur la navette (35).

8. Installation médicale selon l'une des revendications 1 à 7, le système de reconnaissance de position (41, 43) étant conçu de manière à détecter à l'aide d'un codage (55, 57, 59), en plus de la position de la navette (35), un état de fonctionnement et/ou un type de la navette (35).

9. Procédé d'amarrage d'un dispositif de positionnement (33) sur une navette (35), la navette (35) comprenant un dispositif support (39) et un dispositif porteur (37) du dispositif support (39), ledit procédé comportant les étapes suivantes :
- disposer la navette (35) dans un local (19) d'une installation médicale (10),
- détecter la position de la navette (35) par l'intermédiaire d'un système de reconnaissance de position (41, 43),
- commander le dispositif de positionnement (33) de telle façon que la position détectée de la navette (35) soit utilisée lors de l'amarrage du dispositif de positionnement (33) sur la navette (35),
**caractérisé en ce que**
le système de reconnaissance de position (41, 43) est placé dans une zone de plafond du local (19).

10. Procédé selon la revendication 9,
la position de la navette (35) étant détectée à l'aide d'au moins un premier repère (45), placé à un endroit fixe dans le local, et/ou à l'aide d'au moins un deuxième repère (51), placé sur le dispositif porteur (37), et/ou à l'aide d'au moins un troisième repère (53), placé sur le dispositif support (39).

11. Procédé selon la revendication 9 ou 10,
le système de reconnaissance de position (41, 43) permettant de détecter, en plus de la position de la navette (35), un état de fonctionnement et/ou un type de la navette (35), et la commande du dispositif de positionnement (33) étant adaptée sur cette base.

12. Procédé selon l'une des revendications 9 à 11,
un signal d'avertissement étant émis dans le cas où il serait constaté que la navette (35) se trouve hors de portée du dispositif de positionnement (33).
